Europäisches Patentamt

European Patent Office

Office européen des brevets

(19)

(11) Veröffentlichungsnummer: **0 239 718 B1**

(12) **EUROPÄISCHE PATENTSCHRIFT**

(45) Veröffentlichungstag der Patentschrift: 06.03.91

(51) Int. Cl.5: **A61M 5/162**

(21) Anmeldenummer: **87100175.6**

(22) Anmeldetag: **09.01.87**

(54) Schutzvorrichtung für einen Einstichdorn für Transfusions- oder Infusionsgeräte.

(30) Priorität: **27.03.86 DE 8608414 U**

(43) Veröffentlichungstag der Anmeldung:
**07.10.87 Patentblatt 87/41**

(45) Bekanntmachung des Hinweises auf die
Patenterteilung:
**06.03.91 Patentblatt 91/10**

(84) Benannte Vertragsstaaten:
**AT BE CH DE ES FR GB GR IT LI NL SE**

(56) Entgegenhaltungen:
**FR-A- 1 213 861**
**US-A- 3 135 259**
**US-A- 4 405 047**

(73) Patentinhaber: **B. Braun-SSC AG**
**Gerliswilstrasse 74**
**CH-6020 Emmenbrücke(CH)**

(72) Erfinder: **Lesemann, Egon**
**Lindenberg Strasse 28-30**
**W-3508 Melsungen(DE)**
Erfinder: **Wiegel, Heinz**
**Im Hof 2**
**W-6445 Alheim 1(DE)**
Erfinder: **Gossen, Wolfgang**
**Heideweg 12**
**W-3508 Melsungen(DE)**

(74) Vertreter: **von Kreisler, Alek, Dipl.-Chem. et al**
**Deichmannhaus am Hauptbahnhof**
**W-5000 Köln 1(DE)**

## Beschreibung

Die Erfindung bezieht sich auf eine Schutzvorrichtung für einen Einstichdorn; für Transfusions- oder Infusionsgeräte bestehend aus einer auf den Einstichdorn klemmend aufsteckbaren Kappe.

Schlauch-Rollenklemmen dienen bei bekannten (US-A-3135259, US-A-4405047) Infusions- oder Transfusionsgeräten dazu, die Durchflußmenge des Schlauches zu regulieren, der den Patienten mit einem Flüssigkeitsbehälter verbindet und da eine solche Regulierung immer immer erforderlich ist, wird praktisch jedes Infusions-oder Transfusionsbesteck mit einer Schlauch-Rollenklemme ausgerüstet

Einstichdorne, beispielsweise an Tropfkammern, stellen die Verbindung zwischen einem Flüssigkeitsbehälter und einer zu einem Patienten führenden Schlauchleitung her. Sie sind scharf und spitz und werden vom Hersteller mit einer aufgesteckten gesonderten Kappe geliefert, so daß ihre Spitze bis zur Benutzung geschützt ist und Verletzungsgefahr praktisch nicht besteht. Da die Kappe ein loses Einzelteil bildet, wird sie im allgemeinen weggeworfen, sobald der Einstichdorn seine Bestimmung erfüllt. Es hat sich jedoch gezeigt, daß die ungeschützten Einstichdorne der Transfusions- oder Infusionsgeräte auf dem Entsorgungsweg problematisch sind, weil die Geräte in Kunststoffsäkken transportiert werden, durch die die Spitzen der Einstichdorne hindurch treten, wodurch es zu Verletzungen bzw. Vergiftungen des Entsorgungspersonals kommen kann. Dies ist ein wesentlicher Mangel, den es zu beseitigen gilt.

Der Erfindung liegt die Aufgabe zugrunde, eine Schutzvorrichtung für einen Einstichdorn so auszubilden, daß sie auch nach der Benutzung des Transfusions- oder Infusionsgerätes verfügbar ist, so daß auf dem Entsorgungsweg keine Verletzungsgefahr für das Personal besteht.

Diese Aufgabe wird erfindungsgemäß dadurch gelöst, daß die Kappe in Form einer Röhre auf der Außenseite des aus Boden- und Seitenwänden gebildeten länglichen Gehäuses einer Schlauch-Rollenklemme ausgebildet ist.

Durch Integrierung der Schutzvorrichtung für einen Einstichdorn in die Schlauch-Rollenklemme wird daher erreicht, daß auch nach der Benutzung des Transfusions- oder Infusionsbestecks eine Schutzvorrichtung für den Einstichdorn zur Verfügung steht, so daß dessen Spitze auf dem Entsorgungsweg das Personal nicht mehr gefährden kann. Die in die Schlauch-Rollenklemme integrierte röhrenförmige Kappe für den Einstichdorn entlastet im übrigen das Personal von dem Zubehör in Form einer losen kleinen Kappe, die bei dem Aufbau eines Infusions- oder Transfusionsgerätes zusätzlich gehandhabt werden muß, was z.B. auf Intensivstationen, auf denen es auf Geschwindigkeit bei der Patientenversorgung ankommt, nachteilig sein kann.

In vorteilhafter Ausgestaltung der Erfindung ist vorgesehen, daß die Röhre auf der Außenseite des Bodens des Gehäuses angeordnet ist und sich über wenigstens einen Teil seiner Länge erstreckt. Die Röhre verläuft parallel zu dem Gehäuse und dessen Boden und entsprechend ist auch der in die Röhre klemmend eingesetzte Einstichdorn zu der Anordnung der Schlauch-Rollenklemme parallel. Die Röhre kann im Querschnitt Hufeisenform aufweisen und der Boden des Gehäuses die gerade Wand der Röhre bilden. Die Hufeisenform der Röhre begünstigt auf einfache Weise den Reibungsschluß zwischen Röhreninnenwand und Einstichdorn, ohne daß enge Toleranzen zwischen beiden Teilen eingehalten werden müssen. Auch sind herstellungsmäßige Erleichterungen hierdurch gegeben.

Alternativ kann die Röhre im Querschnitt im wesentlichen Kreisform aufweisen. In diesem Falle ist vorteilhafterweise die Außenwand der Röhre geschlitzt. Der Längsschlitz, der im nicht benutzten Zustand der Röhre mehr oder weniger geschlossen ist, öffnet sich beim Einstecken des Einstichdorns und hält durch seine federnde Wirkung den Einstichdorn fest in der Röhre.

Die Länge und der Durchmesser der mit dem Gehäuse der Schlauch-Rollenklemme kombinierten Kappe in Form einer Röhre können so bemessen sein, daß sie nur den Spitzenteil des Einstichdornes oder auch ihren verdickten Stutzenteil aufnimmt, der den Übergang des Einstichdorns zu einer Tropfkammer bildet.

In der Zeichnung sind Ausführungsbeispiele der Erfindung schematisch dargestellt.

Es zeigen:

Fig. 1  einen Längsschnitt des Gehäuses einer Schlauch-Rollenklemme mit einer Ausführungsmöglichkeit der integrierten Kappe in Form einer Röhre,

Fig. 2  eine Ansicht der Anordnung nach Figur 1 in Richtung des Pfeiles A,

Fig. 3  einen Längsschnitt des Gehäuses einer Schlauch-Rollenklemme mit einer zweiten Ausführungsmöglichkeit der integrierten Kappe in Form einer Röhre,

Fig. 4  eine Ansicht der Anordnung nach Figur 3 in Richtung des Pfeiles B,

Fig. 5  einen Längsschnitt des Gehäuses einer Schlauch-Rollenklemme mit einer dritten Ausführungsmöglichkeit der integrierten Kappe in Form einer Röhre und

Fig. 6  eine Ansicht der Anordnung nach Figur 5 in Richtung des Pfeiles C.

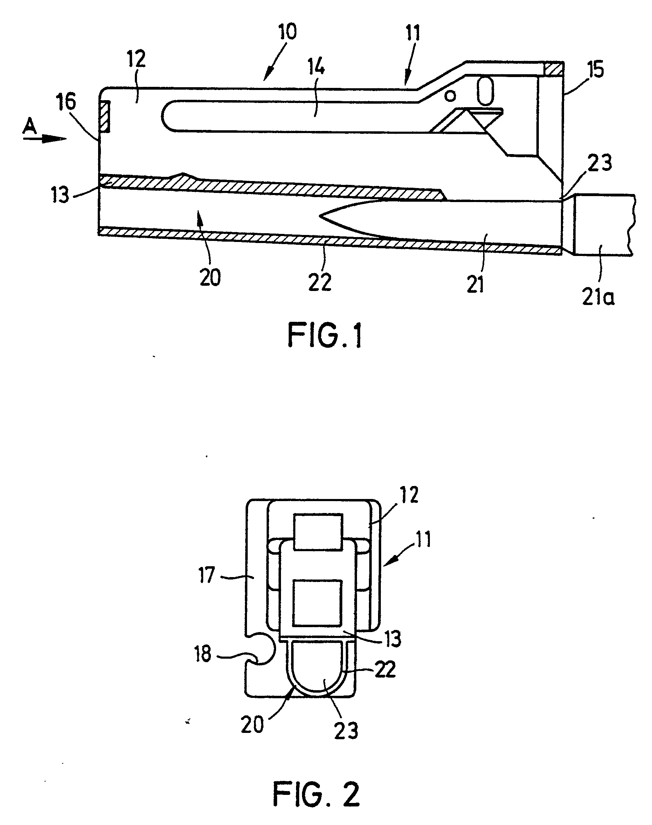

Das Gehäuse 11 einer Schlauch-Rollenklemme 10 besteht im wesentlichen aus zwei zueinander parallelen Seiten wänden 12 und einem Boden 13, auf dem ein nicht gezeichneter Schlauch eines Infusions- oder Transfusionsgerätes liegt, dessen Durchflußmenge mit Hilfe einer ebenfalls nicht gezeigten Rolle reguliert werden soll, die in Nuten 14 in den Seitenwänden 12 längsverschiebbar geführt ist. Da wenigstens die Innenfläche des Bodens 13 in bezug auf die Nuten 14 schräg verläuft, wird der Schlauch bei der Bewegung der Rolle (in Figur 1 nach links) zunehmend zusammengequetscht und der Durchfluß gedrosselt. Das rechte breitere Ende des Gehäuses 11 wird nachfolgend als vorderes Ende 15 bezeichnet. Das gegenüberliegende Ende ist entsprechend das hintere Ende 16 des Gehäuses 11. Am vorderen Ende 15 des Gehäuses 11 ist auf einer Seite eine Leiste 17 ausgebildet, die eine teilkreisförmige Öse 18 aufweist, mit deren Hilfe die Schlauch-Rollenklemme 10 im Nichtbenutzungszustand an den Schlauch angeklemmt wird, ohne ihn zu verformen.

Der Boden 13 des Gehäuses 11 ist auf der Außenseite gerade und eben und er bildet die gerade Oberwand einer im Querschnitt hufeisenförmigen Röhre 20 zur Aufnahme eines im Querschnitt kreisförmigen, spitzen Einstichdornes 21 eines Transfusions- oder Infusionsgerätes nach der Art einer Schutzkappe. Die U-förmig gebogene Außenwand 22 der Röhre 20 erstreckt sich gerade vom vorderen Ende 15 zum hinteren Ende 16 des Gehäuses 11 und die Röhre 20 ist an beiden Enden offen. Die Einstecköffnung 23 der Röhre 20 befindet sich am vorderen Ende 15 des Gehäuses 11 und durch diese Einstecköffnung 23 wird die scharfe Spitze des Einstichdornes 21 in die Röhre 20 eingeschoben, bis sie zwischen dem zur Einstecköffnung 23 zurückgesetzten Boden 13 und der Außenwand 22 klemmend gehalten ist, so daß sie nicht selbständig herausrutschen kann. Sowohl im Neuzustand als nach der Benutzung des Transfusions- und Infusionsbestecks kann der Einstichdorn 21 geschützt in der Röhre 20 untergebracht sein, so daß er zu keiner Zeit eine Verletzungsgefahr für das Personal bedeutet.

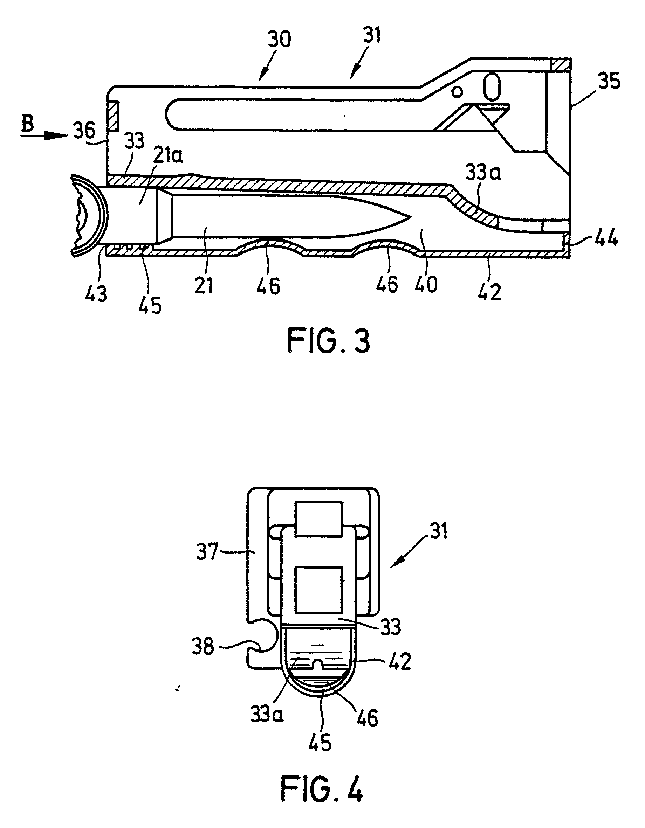

Eine weitere Ausführungsmöglichkeit der Schutzvorrichtung für den Einstichdorn 21 ist in den Figuren 3 und 4 dargestellt. In diesem Falle ist das Gehäuse 31 einer Schlauch-Rollenklemme 30 an der Außenseite seines Bodens 33 mit der Kappe in Form einer Röhre 40 ausgestattet, deren Einstecköffnung 43 sich am hinteren Ende 36 des Gehäuses 31 befindet. Bei diesem Beispiel ist der Boden 33 des Gehäuses 31 weit bis zum vorderen Ende 35 des Gehäuses 31 durchgezogen und verläuft im Bereich dieses vorderen Endes 35 nach außen muldenförmig gekrümmt, wodurch sich eine Verengung der Röhre 40 an diesem vorderen Ende 35 ergibt, wenn die Außenwand 42 der im Querschnitt hufeisenförmigen Röhre 40 in Achsrichtung des Gehäuses 31 gerade ist. Die muldenförmige Krümmung des Bodens 33 dient der Erleichterung des Einführens der nicht gezeichneten Rolle der SchlauchRollenklemme bei ihrer Montage. Eine Wand 44 verschließt das eine Ende der Röhre 40, die bei diesem Ausführungsbeispiel den kompletten Einstichdorn 21 einschließlich seines Stutzenteiles 21a aufnimmt. Der Stutzenteil 21a des Einstichdornes 21 ist im wesentlichen passend in den Mündungsbereich der Röhre 40 eingesetzt, wobei zur Verbesserung der Haltewirkung gekrümmte flache Querrippen 45 in dem Mündungsbereich vorgesehen sind, die an den Stutzenteil 21a angreifen. Ferner sind vorzugsweise zwei umlaufende Fingervertiefungen 46 in der Außenwand 42 der Röhre 40 ausgebildet, die ein Verrutschen des Gehäuses 31 in der Hand des Anwenders verhindern sollen und die außerdem die Spitze des Einstichdorns 21 innerhalb der Röhre 40 abstützen, um seinen Zusammenhalt mit dem Gehäuse 31 zu verbessern. An dem vorderen Ende 35 des Gehäuses 31 ist auf einer Seite eine Leiste 37 angeordnet, die eine offene Öse 38 aufweist, die der Öse 18 des Beispiels der Figuren 1 und 2 entspricht und dem gleichen Zweck dient.

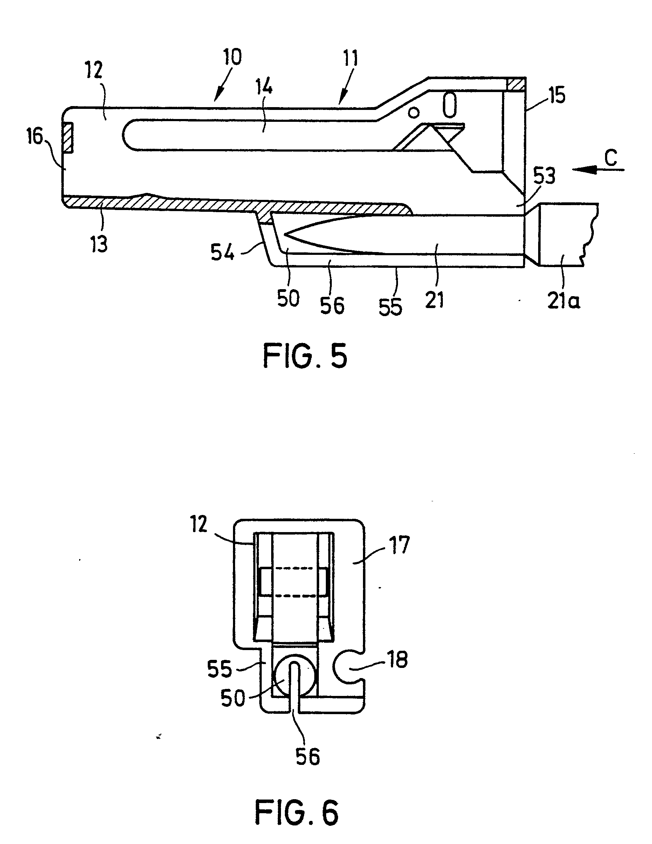

Ein drittes Ausführungsbeispiel wird von den Figuren 5 und 6 veranschaulicht. Es handelt sich hierbei um eine alternative Integrierung einer röhrenförmigen Kappe in das Gehäuse der Schlauch-Rollenklemme 10 der Figuren 1 und 2. Hierbei erstreckt sich eine Röhre 50 nur über den vorderen Teil des Bodens 13, der selbst mit Abstand zu dem vorderen Ende 15 des Gehäuses 11 endet, um die Einführung einer Rolle in das Gehäuse 11 zu ermöglichen. Die Röhre 50 ist aus einer etwa rechteckigen Außenwand 55 und einer Rückwand 54 gebildet, die etwa in der Mitte des Bodens 13 von diesem nach außen absteht und in Richtung des vorderen Endes 15 des Gehäuses 11 schräg geneigt ist. Die Außenwand 55 ist an der Unterseite durchgehend längsgeschlitzt und der Längsschlitz 56 erstreckt sich bis in die Rückwand 54 hinein. Er endet mit geringem Abstand zur Außenfläche des Bodens 13. Bei diesem Beispiel befindet sich die Einstecköffnung 53 am vorderen Ende 15 des Gehäuses 11 und die Röhre 50 nimmt nur die Spitze des Einstichdornes 21 auf und nicht den verdickten Stutzenteil desselben. Der Hohlraum der Röhre 50 ist zur Anpassung an die Spitze des Einstichdornes 21 zylindrisch ausgebildet. Der Längsschlitz 56, der im nicht benutzten Zustand mehr oder weniger geschlossen ist, öffnet sich beim Einstecken des Einstichdornes 21 in die Röhre 50 und hält durch seine federnde Wirkung den Einstichdorn 21 in der Röhre 50 fest, so daß er nicht versehentlich herausrutschen kann.

## Ansprüche

1. Schutzvorrichtung für einen Einstichdorn, für Transfusions- oder Infusionsgeräte bestehend aus einer auf den Einstichdorn klemmend aufsteckbaren Kappe,
   **dadurch gekennzeichnet,**
   daß die Kappe in Form einer Röhre (20) auf der Außenseite des aus Boden- und Seitenwänden (13,12) gebildeten länglichen Gehäuses (11) einer Schlauch-Rollenklemme (10) ausgebildet ist.

2. Schutzvorrichtung nach Anspruch 1, dadurch gekennzeichnet, daß die Röhre (20) auf der Außenseite des Bodens (13) des Gehäuses (11) angeordnet ist und sich über wenigstens einen Teil seiner Länge erstreckt.

3. Schutzvorrichtung nach Anspruch 1 oder 2, dadurch gekennzeichnet, daß die Röhre (20;40) im Querschnitt Hufeisenform aufweist und daß der Boden (13;33) des Gehäuses (11;31) die gerade Wand der Röhre bildet.

4. Schutzvorrichtung nach Anspruch 1 oder 2, dadurch gekennzeichnet, daß die Röhre (50) im Querschnitt im wesentlichen Kreisform aufweist.

5. Schutzvorrichtung nach Anspruch 4, dadurch gekennzeichnet, daß die Außenwand (55) der Röhre (50) längsgeschlitzt ist.

6. Schutzvorrichtung nach einem der Ansprüche 1 bis 5, dadurch gekennzeichnet, daß die Einstecköffnung (23;43;53) der Röhre (20;40;50) mit einem Ende (15;36) des Gehäuses (11;31) bündig verläuft.

7. Schutzvorrichtung nach einem der Ansprüche 1 bis 6, dadurch gekennzeichnet, daß in der Außenwand (42) der Röhre (40) umlaufende Fingervertiefungen (46) ausgebildet sind.

## Claims

1. A shielding device for a piercing needle for transfusion or infusion devices, consisting of a cap which may be jammingly plugged onto said piercing needle, characterized in that said cap is formed as a tube (20) on the outside of the elongated case (11) of a roller hose clip (10), said case being formed by bottom and side walls (13, 12).

2. The shielding device of claim 1, characterized in that said tube (20) is arranged on the outside of the bottom (13) of said case (11) and extends at least over a portion of the length thereof.

3. The shielding device of claim 1 or 2, characterized in that said tube (20; 40) is horseshoe-shaped in cross section and that the bottom (13; 33) of the case (11; 31) forms the straight wall of said tube.

4. The shielding device of claim 1 or 2, characterized in that said tube (50) is substantially circular in cross section.

5. The shielding device of claim 4, characterized in that the outer wall (55) of said tube (50) is longitudinally slotted.

6. The shielding device of one of claims 1 to 5, characterized in that the insert opening (23; 43; 53) of said tube (20; 40; 50) is flush with one end (15; 36) of said case (11; 31).

7. The shielding device of one of claims 1 to 6, characterized in that circumferential indentations (46) for the fingers are provided in the outer wall (42) of said tube (40).

## Revendications

1. Dispositif de protection pour un perforateur destiné à des appareils de transfusion ou d'infusion, composé d'une calotte à enfoncer par serrage sur le Peforateur, caractérisé en ce que la calotte est réalisée sous forme d'une tubulure (20) sur le côté extérieur du boîtier longitudinal (11), constitué par des parois de fond et de côté (13, 12), d'une pince à rouleau pour tuyau (10.

2. Dispositif de protection selon la revendication 1, caractérisé en ce que la tubulure (20) est disposée sur le côté extérieur du fond (13) du boîtier (11) et s'étend sur au moins une partie de sa longueur.

3. Dispositif de protection selon la revendication 1 ou 2, caractérisé en ce que la tubulure (20; 40) présente en coupe transversale une forme de fer à cheval et en ce que le fond (13; 33) du boîtier (11 ; 31) constitue la paroi droite de la tubulure.

4. Dispositif de protection selon la revendication 1 ou 2, caractérisé en ce que la tubulure (50) présente en coupe transversale une forme sensiblement circulaire.

5. Dispositif de protection selon la revendication 4, caractérisé en ce que la paroi extérieure (55) de la tubulure (50) est fendue longitudinalement.

6. Dispositif de protection selon l'une des revendications 1 à 5, caractérisé en ce que l'orifice d'enfoncement (23 ; 43 ; 53) de la tubulure (20 ; 40 ; 50) affleure à une extrémité (15; 36) du boîtier (11; 31).

7. Dispositif de protection selon l'une des revendications 1 à 6, caractérisé en ce que des évidements périphériques en forme de doigts (46) sont réalisés dans la paroi extérieure (42) de la tubulure (40).

FIG.1

FIG. 2

6

**B** → 36

30    31    35

33    21a    33a

43    45    21    46    46    40    42    44

# FIG. 3

37    31

38    33

33a    42    46

45

# FIG. 4

FIG. 5

FIG. 6